# EUROPEAN PATENT APPLICATION

(11) **EP 4 663 014 A1**
(43) Date of publication of application: **17.12.2025**
(21) Application number: 24753355.7
(22) Date of filing: 06.02.2024
(51) Int. Cl.: A01K 67/027, C12N 5/09, G01N 33/15, G01N 33/50

(54) **CANCER MODEL ANIMAL AND METHOD FOR PRODUCING SAME**

(30) Priority: 06.02.2023 JP 2023016015
(71) Applicant: Orbio Corporation, Kyoto-shi, Kyoto 602-0841 (JP)
(72) Inventor: ONO, Koki, Hirosaki-shi, Aomori 036-8560 (JP); GOTO, Shintaro, Hirosaki-shi, Aomori 036-8560 (JP); KIJIMA, Hiroshi, Hirosaki-shi, Aomori 036-8560 (JP); YAMADA, Katsuya, Hirosaki-shi, Aomori 036-8560 (JP)
(74) Representative: Mewburn Ellis LLP
(86) International application number: PCT/JP2024/003930
(87) International publication number: WO 2024/166912

(57) **Abstract**

Provided are: a cancer model animal in which a malignant tumor corresponding to a pathological image of human invasive cancer or metastasis has been formed or can be formed, despite the use of transplantation under the skin or into the abdominal cavity; and a method for producing the cancer model animal. One aspect of the present invention is: a cancer model animal in which tumor cells that grow in a culture medium containing L-glucose are transplanted under the skin or into the abdominal cavity: a sarcoma model animal in which tumor cells that have been transplanted under the skin or into the abdominal cavity invade muscles; a cancer model animal in which tumor cells, such as adenocarcinoma cells, that have been transplanted under the skin or into the abdominal cavity show metastasis; or a cancer model animal in which tumor cells, such as adenocarcinoma cells, that have been transplanted under the skin or into the abdominal cavity show anomaly of function of orthotopic cells. Another aspect of the present invention is a method for producing a cancer model animal, the method including a step for transplanting a cell composition under the skin or into the abdominal cavity of an animal, the cell composition containing tumor cells that grow by culturing in a culture medium containing L-glucose and being used for producing a cancer model animal by transplanting the cell composition under the skin or into the abdominal cavity of the animal.

## Description

### TECHNICAL FIELD

The present invention relates to a cancer model animal and to a method for creating the same.

### BACKGROUND ART

When a newly discovered or developed agent is found to inhibit tumor growth in vitro, it should be demonstrated whether the agent is also effective in inhibiting tumor growth in vivo. A general procedure for such demonstration includes first using a model animal to determine whether the agent is effective in inhibiting tumor growth (namely a non-clinical trial) and then verifying the results in humans (namely a clinical test). Appropriate selection or creation of a model animal for the animal experiment prior to human trials plays a critically important role in the development of new antitumor agents. A known example of such a model animal includes a lung cancer-transplanted model animal, which is created by intratracheally injecting lung cancer cells into a non-human animal (Patent Document 1).

### Citation List

### Patent Document

Patent Document 1: Japanese Unexamined Patent Application, Publication No. 2007-274950

### DISCLOSURE OF THE INVENTION

### Problems to be Solved by the Invention

The survival rate of cancer patients (patient outcome) will significantly decrease as the cancer invades deeper tissues and develops metastasis. Unfortunately, low-survival-rate cancers are generally difficult to detect early and can be found to already develop metastasis at their initial diagnosis. Such cancers are impossible to remove by surgery, or even when treated by surgery in the case of no clear metastasis found, such cancers may be found to recur or develop metastasis during the post-surgery period.

To address such challenges, two approaches are pursued: first, enabling earlier diagnosis for cancer, for example, by developing methods for detecting cancer at the cellular level at an earlier stage or developing biomarkers correlated with cancer development; and second, developing a chemotherapy, a radiation therapy, or any other treatment technology that can reduce the aggressiveness of advanced cancer, recover patients to the extent that the cancer is controllable, or improve the prognosis.

Taking chemotherapy as an example, however, low-survival-rate cancers may often recur and become therapy-resistant even when they have appeared to be less aggressive after aggressive treatment intervention using anticancer agents recommended as the first-line treatment. Some new molecular target drugs and immune checkpoint inhibitors targeting the immune system have also been put to practical use, but only limited patients can benefit from them. In particular, immune-targeted agents can have diverse side effects, which are difficult to manage in clinical practice and may lead to strong life-threatening side effects, and thus should not be used as the first-line treatment in the current situation where no information is available in advance about whether they are beneficial or not.

In fact, guidelines for treatment of pancreatic cancer and bile duct cancer, known to have the lowest survival rate, and other low-survival-rate cancers still recommend, as the first-line treatment, one or a combination of two or more of gemcitabine, 5-FU, platinum-based formulations, paclitaxel, and other traditional anticancer agents that have low cancer specificity and strong side effects against normal cells (Drug combinations used in pancreatic cancer, National Cancer Institute). Although a large number of various traditional therapeutic combinations have been tried and shown some improvement in short-term survival, it still remains a challenging task to conquer refractory cancers of which the 10-year survival rate still remains very low (President Biden, "Fact Sheet: President Biden reignites cancer moonshot to end cancer as we know it." The white house. February 02, 2022).

In that sense, basic research is the key to enabling breakthrough technological innovations in all areas of cancer detection, diagnosis, and treatment. For example, if a newly discovered or developed agent is found to inhibit tumor growth in vitro, the next step will be to demonstrate that it is also effective in inhibiting tumor growth in vivo. A general procedure for such demonstration includes first using a model animal to determine whether the agent is effective in inhibiting tumor growth (namely a non-clinical trial) and then verifying the results in humans (namely a clinical test). The verification in humans involves a large-scale process that includes material stability tests and then several phases of clinical trials. Thus, if the drug is not demonstrated to have sufficient efficacy at the human verification stage, the drug development company will suffer a serious loss. Therefore, appropriate selection or creation of an animal model for the animal experiment prior to human trials plays a critically important role in the development of new antitumor agents or other cancer therapies. An excellent model animal for cancer is also necessary for the development of breakthrough technologies for cancer detection or diagnosis. The development of such an excellent model animal for cancer plays an extremely important role in innovating technologies for cancer detection, diagnosis, and treatment. On the other hand, it may be no exaggeration to say that inappropriate animal models can be a major factor that hinders technological innovations for cancer detection, diagnosis, and treatment at an early research stage.

Cancer model animals, which are experimental animals with transplanted tumor cell lines, have been used for many years to develop cancer detection technologies or to roughly evaluate and screen various therapies for tumor growth-inhibiting efficacy at an early stage. In particular, methods for producing tumor-bearing mice with subcutaneously transplanted cancer cells are still widely used at present as simple methos for model animal production (Stribbling, SM. et al., Nat. Protoc. 17: 2108-2128, 2022). In such tumor-bearing animals created by subcutaneous transplantation of tumor cell lines, however, it is not easy to induce such a phenomenon as cancer invasion or metastasis although the tumor can grow in the transplanted subcutaneous site. Since the subcutaneous tissue differs from the organ from which the necessary cancer cells are derived, what is called orthotopic transplantation is also performed, which involves transplanting cancer cells into an organ corresponding to the origin of tumor. In such orthotopic transplantation, however, the engraftment of tumor cell lines in the tissue is generally low.

Therefore, to promote the engraftment of transplanted cells, it is common to add, during transplantation, a mixture of extracellular matrices (such as Matrigel^{™}, Thermo Fisher Scientific) derived from tissues or species completely different from the organ from which the necessary cancer cells are derived (Stribbling, SM. et al., Nat. Protoc. 17: 2108-2128, 2022). Matrigel^{™} includes molecules prepared through extraction from a basement membrane reconstituted from isolated EHS mouse sarcoma cells. Thus, the orthotopic transplantation with such a molecule as Matrigel^{™} added as a necessary cofactor for transplantation cannot be denied as being unnatural in terms of the artificial microenvironment introduction. The orthotropic transplantation also requires more complex surgical procedures than the subcutaneous or intraperitoneal transplantation and has a difficulty level and a success rate that will increase and decrease, respectively, as the required factors such as the region and depth for the transplantation into the corresponding organ become more strict. Thus, the orthotropic transplantation is not suitable for convenient applications for the development and screening of diagnostic or therapeutic methods and other purposes.

To actually evaluate the tumor-inhibiting effect of antitumor substances, it is necessary to repeatedly conduct a series of non-clinical trials including determination of concentration dependency, selection of administration schedules, comparison with standard therapeutic drugs to verify superiority, and determination of the relationship between the drug effect and the pathological evaluation of cancer. Such a series of non-clinical trials requires using a large number of animals and performing long-term experiments, which realistically requires a lot of labor and allows convenient animal models such as subcutaneously transplanted animals to still continue to be used for initial screening despite their drawbacks.

It is recognized that a lot of attention should be paid not only to the transplantation site but also to the cells to be transplanted (Stribbling, SM. et al., Nat. Protoc. 2022). For example, it has been pointed out that, in the course of many years of repeated passages using a special liquid medium and an artificial environment, commercially available tumor cell lines can preferentially become more adapted to the culture environment even though they had the characteristics of cancer cells at the time when extracted from a patient's organ.

Thus, cancer patient's tumor tissues, metastatic site tissues, or blood sample cells are used directly without passage or after only a limited number of passages, or patient-derived xenograft (PDX) mouse models are used, which are immunodeficient mice with such cancer patient's tissues or cells transplanted in the subcutaneous site (Aparicio, S. et al., Nat. Rev. Cancer 15: 311-316, 2015). Unfortunately, such a method takes a long time until establishment and has drawbacks such as difficulties in scaling up and maintenance and availability for only a limited number of researchers. It is also recognized that even samples extracted from the same patient's cancer tissue may exhibit different cellular characteristics depending on the site from which they are obtained. In extreme cases, even if a drug effective in inhibiting a specific part of a patient's tumor is developed successfully, it may be ineffective against cancer in a different site of the patient or against tumors in a different patient, which raises concerns about reproducibility. As described above, the tumor-bearing mouse model created using tumor cell lines and the PDX each have their own advantages and disadvantages and appear to be on opposite sides.

However, some low-survival-rate cancers are recognized to share common features. Some cancer cells share pathologically similar cell morphologies and features, such as: neuroendocrine tumors, which are known for their strong resistance to treatment regardless of substantially different origins of tumor including gastrointestinal tract, pancreas, and prostate; gynecologic cancers such as ovarian cancer; and clear cell carcinoma of renal cancer, which is known for its poor response to chemotherapy and poor prognosis. While derived from different origins, some cancers such as sarcomas also share the common feature of invading muscles or bones.

In view of the above, if there is a convenient and highly-useful cancer model animal that can be produced through an easy transplantation procedure and can show invasion or distant metastasis feature with no aid of a cofactor such as an extracellular matrix, it will be expected to be useful for developing and evaluating methods for detection, diagnosis, and treatment of poor prognosis cancers.

It is an object of the present invention to provide a cancer model animal that has or is capable of developing a malignant tumor comparable to a human invasive cancer or metastasis feature although created through subcutaneous or intraperitoneal transplantation; and a method for creating such a cancer model animal.

### Means for Solving the Problems

The inventors have conducted intensive research to provide a solution to the problem described above. As a result, the inventors have completed the present invention based on findings that a specific cancer model animal created using L-glucose can provide a solution to the problem. Specifically, the present invention provides the following aspects.
(1) A cancer model animal comprising subcutaneously or intraperitoneally transplanted tumor cells that proliferate by culturing with an L-glucose-containing medium.
(2) A sarcoma model animal comprising subcutaneously or intraperitoneally transplanted tumor cells invading a muscle.
(3) The sarcoma model animal according to (2), wherein the muscle is a skeletal muscle.
(4) A caner model animal comprising subcutaneously or intraperitoneally transplanted tumor cells showing metastasis, wherein the tumor cells are selected from the group consisting of adenocarcinoma cells, neuroendocrine tumor cells, and sarcoma cells.
(5) The cancer model animal according to (4), wherein the tumor cells are adenocarcinoma cells, and wherein the metastasis is metastasis to a lymph node, a lung, or an intra-abdominal organ or accumulation in a renal hilum.
(6) The cancer model animal according to (5), wherein the adenocarcinoma cells are lung cancer-derived tumor cells.
(7) The cancer model animal according to (5), wherein the intra-abdominal organ is liver or spleen.
(8) The cancer model animal according to (4), wherein the tumor cells are neuroendocrine tumor cells and wherein the metastasis is metastasis to a lymph node, an intra-abdominal organ, or a peritoneal membrane.
(9) The cancer model animal according to (8), wherein the neuroendocrine tumor cells are insulinoma cells.
(10) The cancer model animal according to (8), wherein the intra-abdominal organ is liver.
(11) A cancer model animal comprising subcutaneously or intraperitoneally transplanted tumor cells showing anomaly in orthotopic cell function, wherein the tumor cells are selected from the group consisting of adenocarcinoma cells, neuroendocrine tumor cells, and sarcoma cells.
(12) The cancer model animal according to (11), wherein the adenocarcinoma cells are lung cancer-derived tumor cells.
(13) The cancer model animal according to (11), wherein the neuroendocrine tumor cells are insulinoma cells.
(14) The cancer model animal according to (11), wherein the tumor cells show anomaly in orthotopic cell function within 3 days after transplantation.
(15) The cancer model animal according to (11), wherein the tumor cells show anomaly in orthotopic cell function when the number of the tumor cells is 5.0 × 10⁵ or less.
(16) A cell composition comprising tumor cells that proliferate by culturing with an L-glucose-containing medium, wherein
   the cell composition is subcutaneously or intraperitoneally transplanted into an animal and is used to create a cancer model animal.
(17) A method for creating a cancer model animal, the method comprising subcutaneously or intraperitoneally transplanting the cell composition according to (16) into an animal.
(18) A method of screening substances that have anticancer effect, the method comprising:
   a step of administering a test substance to the cancer model animal according to any one of (1) to (15);
   a step of evaluating tumor cell growth or tumor cell metastasis after starting the administration of the test substance; and
   a step of comparing a result of the evaluation in the evaluating step with a result of evaluation of tumor cell growth or tumor cell metastasis in a model animal to which the test substance was not administered.

### Effects of the Invention

The present invention provides a cancer model animal that has or is capable of developing a malignant tumor comparable to a human invasive cancer or metastasis feature although created through subcutaneous or intraperitoneal transplantation; and a method for creating such a cancer model animal.

### BRIEF DESCRIPTION OF THE DRAWINGS

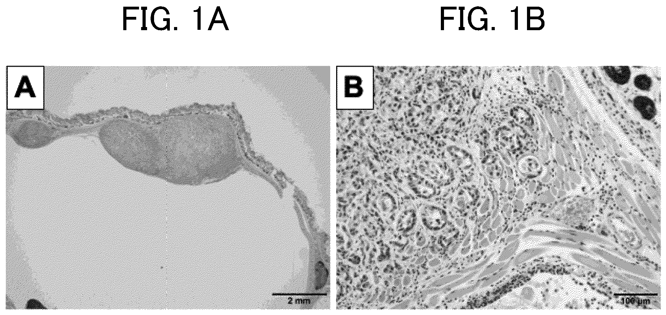

FIG. 1A is a photograph showing a low-magnification image of cancer that is caused by intraperitoneal transplantation of human lung cancer-derived tumor cells A549 cultured with L-glucose and clearly invades the abdominal muscle; and FIG. 1B is a photograph showing a high-magnification image of the tumor in FIG. 1A.

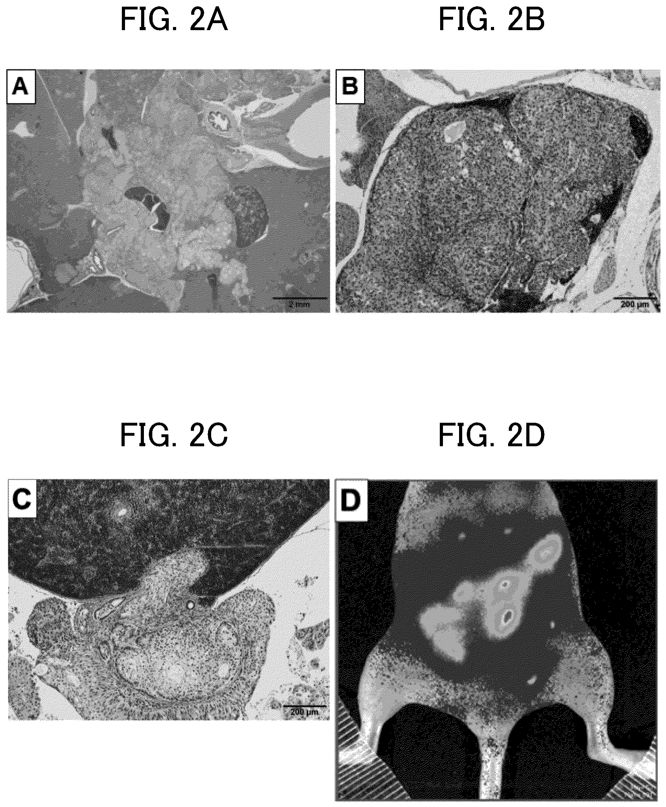

FIG. 2A is a photograph showing liver metastasis of human lung cancer-derived tumor cells A549 cultured with L-glucose; FIG. 2B is a photograph showing lymph node metastasis of the tumor cells; FIG. 2C is a photograph showing spleen metastasis of the tumor cells; and FIG. 2D is a photograph showing the tumor cells invading each organ, which were created by transplantation of luciferase gene-transduced A549 cells and visualized outside the body using a whole-body imager (IVIS Lumina Imaging System, Caliper). It is shown that the tumor widely invades not only into the liver, spleen, and lymph node but also to the abdominal cavity.

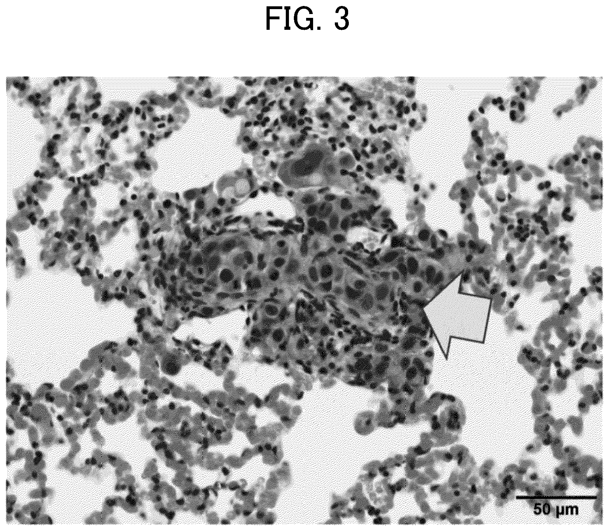

FIG. 3 is a photograph showing cancer that was observed in the lung after intraperitoneal transplantation of human lung cancer-derived tumor cells A549 cultured with L-glucose. The arrow indicates the cancer. It is shown that the cancer has undergone vascularization and settled in the lung.

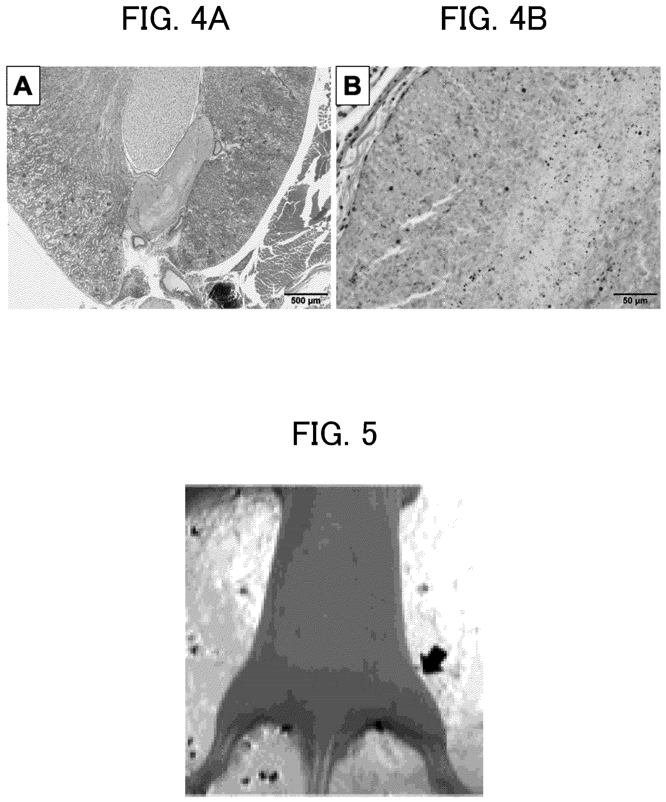

FIG. 4A is a photograph showing a low-magnification image of a giant thrombus that was observed in the renal hilum vein of the kidney; and FIG. 4B is a photograph showing an enlarged image of FIG. 4A.
FIG. 5 is a photograph showing a mouse 7 days after the subcutaneous transplantation of L-glucose-cultured MIN6 cells into the right hindlimb. The arrow indicates the subcutaneous transplantation site. The tumor cell-transplanted subcutaneous site does not show swelling that is normally observable in subcutaneously transplanted animal models.

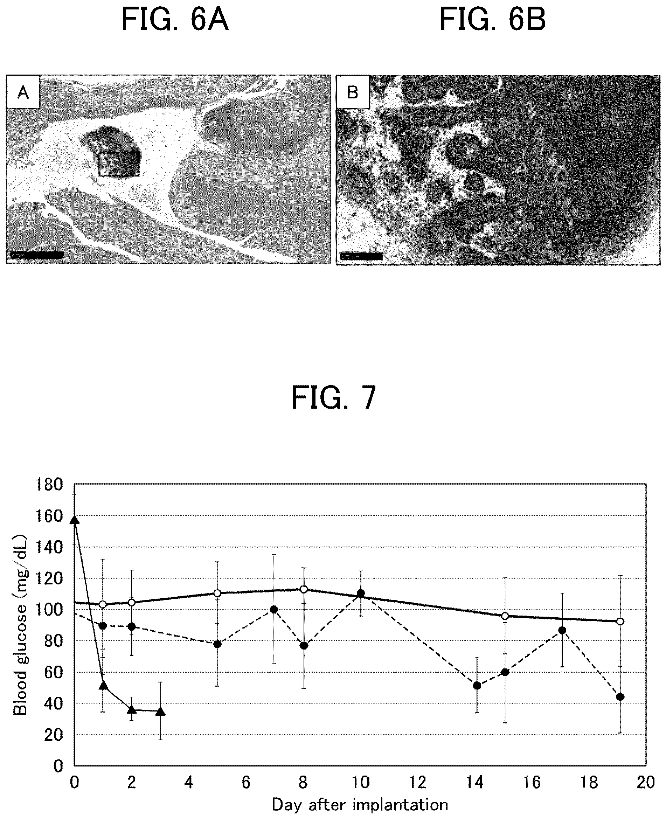

FIG. 6A is a low-magnification photograph showing an HE-stained image of a sarcoma resulting from invasion of the tumor cells (transplanted subcutaneously into the hindlimb of the mouse shown in FIG. 5) into a central portion of the muscle without accumulating in the subcutaneous site; and FIG. 6B is a high-magnification photograph of the sarcoma, which shows very aggressive histopathological features.
FIG. 7 is a graph showing that mice having subcutaneously-transplanted, L-glucose-cultured insulinoma cells exhibited a statistically significant reduction in blood sugar level (the filled triangle) as compared with mice having subcutaneously-transplanted, normally-D-glucose-cultured insulinoma cells (the filled circle) or mice in the normal control group (the hollow circle). It is clearly shown that the subcutaneously-transplanted, L-glucose-cultured insulinoma cells caused the mice to show a statistically significant reduction in blood sugar level already on day 1 after transplantation as compared with the subcutaneously-transplanted, D-glucose-cultured insulinoma cells; communicated with blood flow; and functioned to secrete insulin.

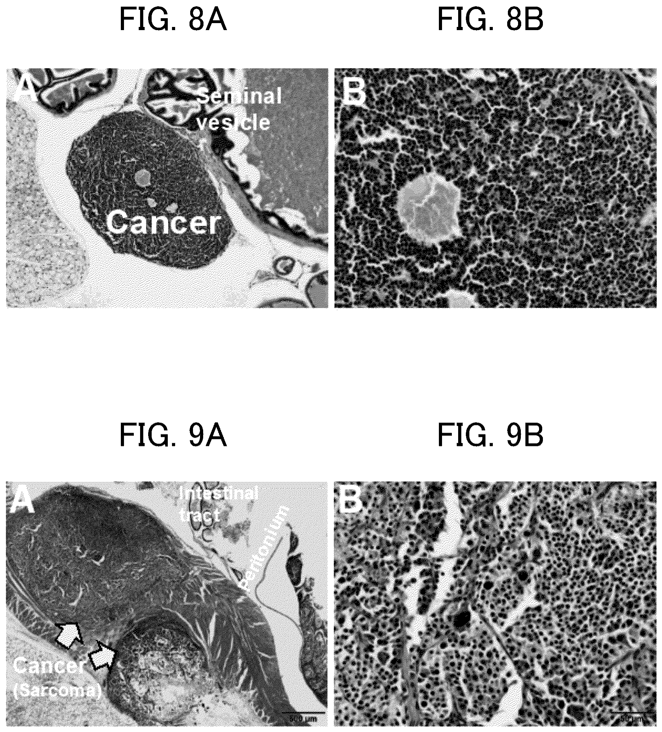

FIG. 8A is a photograph showing an HE-stained image of a tumor that is completely engrafted in the abdominal cavity and has blood supply; and FIG. 8B is a photograph showing an enlarged image of the tumor in FIG. 8A. FIG. 8B shows that the tumor has strong atypia and many mitotic figures and is a considerably high-grade cancer.
FIG. 9A is a photograph showing an HE-stained image of a tumor invading the abdominal muscle. The arrows indicate the tumor. FIG. 9B is a photograph showing an enlarged image of a highly atypical, high-grade, malignant tumor.

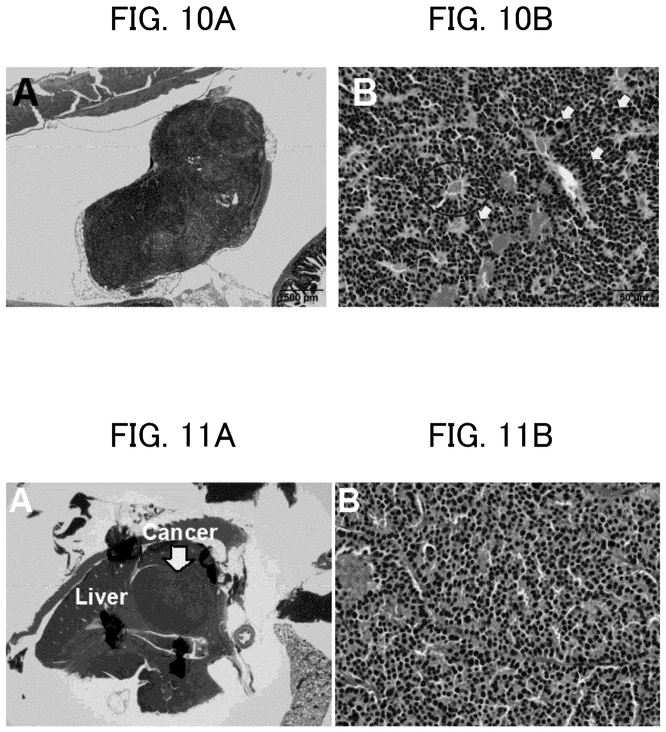

FIG. 10A is a photograph showing an HE-stained image of lymph node metastasis of tumor cells transplanted into the abdominal cavity of a mouse; and FIG. 10B is a photograph showing an enlarged image of the tumor in FIG. 10A.
FIG. 11A is a photograph showing an HE-stained image of liver metastasis of tumor cells transplanted into the abdominal cavity of a mouse; and FIG. 11B is a photograph showing an enlarged image of the tumor in FIG. 11A.

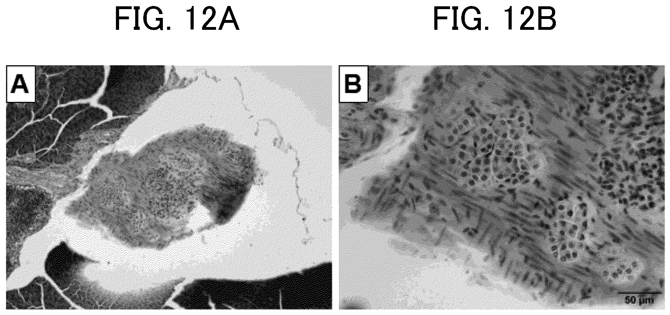

FIG. 12A is a photograph showing an HE-stained image of smooth muscle metastasis of tumor cells transplanted into the abdominal cavity of a mouse; and FIG. 12B is a photograph showing an enlarged image of the tumor in FIG. 12A.

### PREFERRED MODE FOR CARRYING OUT THE INVENTION

Hereinafter, preferred embodiments of the present invention will be described in detail. It should be noted that the embodiments described below are not intended to limit the present invention.

As described above, there is an urgent need for cancer model animals or cancer cell lines that exhibit properties common to those of human cancer, are useful for evaluating antitumor drug activity, and are as simple and easy to use as possible. Thus, the inventors have conducted research based on the following concept, which is completely different from that for PDX.

As mentioned in the "Problem to be Solved by the Invention" section, a reason for why malignant tumor or cancer is feared and should be treated can be said to be that malignant tumor cells can invade or distantly metastasize to a site where they should not normally exist, abnormally proliferate, impair the function of normal tissues, and ultimately cause lethal effects on the body. In contrast, benign tumors, though exhibiting cell proliferation, maintain the original function and morphological features of the proliferative cells and do not invade regions having functions histologically different from those of the tumor cells, which is a key feature for distinguishing benign tumors from malignant tumors. As long as tumor cells reside in their original location while maintaining their morphological and functional features, they can receive nutrient supply via blood flow even when the tumor size is large. In contrast, tumor cells that have invaded regions with functions different from their own functions are expected to face difficulties in receiving nutrients necessary for their growth since they do not have the same features as those of the normal cells in the invasion regions.

Not only cancer cells but all cells require nutrients (or carbon sources) as an energy source for growth. A representative nutrient (or carbon source) available for organisms is D-glucose. So called PET scan is a cancer diagnostic technique that enables cancer imaging based on such organisms' properties (Gambhir, S.S. Nat. Rev. Cancer 2: 683-693, 2002). Specifically, the PET scan includes detecting, from outside the body, the strong cellular uptake of a D-glucose derivative labeled with radioactive ¹⁸F at the 2-position of D-glucose (commonly known as FDG) by positron emission tomography. Such FDG-PET scan is a highly-useful imaging modality that is widely used worldwide for determining, across a wide range of cancer types, whether canter is present and where metastasis occurs. In the diagnostic method based on D-glucose, however, D-glucose is a nutrient utilized not only by cancer cells but also by normal cells surrounding the cancer. Not infrequently, therefore, the cancer detection may be difficult in the brain, which strongly takes up D-glucose, the cancer detection may be interfered with by muscle movement or fat, or inflammation (non-cancer cell anomaly) may be mistaken for cancer.

For the uptake of D-glucose, cancer cells must compete with normal cells, which have an advantage since they can receive the nutrient from blood flow in established tissue structures. Thus, if there are tumor cells capable of surviving and proliferating in such an environment where the nutrient uptake is challenging, they can be said to have a strong invasive potential (an important nature for cancer cells to survive). The inventors have hypothesized that tumor cells capable of taking up, metabolizing, and utilizing, as a growth energy source (or carbon source), a "nutrient that normal cells cannot or almost not utilize" have a high potential to grow, invade, and survive without competition for nutrient uptake with a large number of cells in the invasion site, namely, a high potential to form cancer. The inventors have particularly focused on L-glucose as an example of such a nutrient.

L-glucose is an enantiomer of D-glucose, which is the minimum unit of starch. L-glucose is not or almost not found in nature. It is reported that normal cells cannot take up or metabolize L-glucose (Rudney, H. Science 1940, 92, 112-113). In fact, it is well known that L-glucose is not transported into cells by the glucose transporter, a transmembrane protein used by mammalian cells to take up D-glucose (Ono, K. et al., Cancers 12: 850, 2020).

However, as described below, the inventors have demonstrated that various tumor cells exhibiting malignant features take up L-glucose via a specific mechanism. First, it has been found that 2-NBDLG, an L-glucose analog having a green fluorescent group (NBD) is taken up into many types of malignant tumor cells to cause them to emit fluorescence (Sasaki, A. et al., Human Cell 29: 37-45, 2016). Next, it has been found and reported that the uptake of L-glucose into tumor cells also occur even when the fluorescent group NBD is replaced by a blue one (Coumarin), which means that L-glucose common to both plays an important role, and that the uptake of L-glucose is mediated by a channel-like protein that can be inhibited by apple polyphenol phloretin (Otsuka Y. et al., Org. Lett. 18: 1338-1341, 2016; Yamada, K. et al., US10001487B2; US10509041B2; EP3130596B1; and Japanese Patent No. 6566348). The uptake of L-glucose is also observed in cultured pancreatic tumor cells exhibiting malignant features, cancer tissues of bile duct cancer hamster models, tumor tissues harvested from gastric cancer patients, cells in peritoneal fluid samples collected immediately after laparotomy and before organ resection during surgery for endometrial or ovarian cancer patients, urine from patients with urinary tract cancers such as bladder or ureteral cancer, and sarcoma cells (Yokoyama, H., et al., Human Cell 29: 111-121, 2016; Ogawa, T. et al., Human Cell 34: 634-643, 2021; Yamada, K. et. al., US10551387B2; EP3199638B1; and Japanese Patent Nos. 6670503 and 6406715).

For the investigation of whether L-glucose itself can serve as a nutrient for tumor cells to proliferate, therefore, tumor cells were cultured with a certain amount of L-glucose in place of the same amount of D-glucose, which is usually always added as a nutrient for tumor cell culture (hereinafter, such culture will also be referred to as L-glucose culture). As a result, while many cells died or deteriorated, a small number of cells survived, began to proliferate, and spontaneously formed cell aggregates called spheroids without the use of a special low-adhesion container or Matrigel, which has been reported (Japanese Patent No. 6721868).

L-glucose has four asymmetric carbon atoms that form a steric configuration completely opposite that of D-glucose. L-glucose cannot pass through a glucose transporter that transports D-glucose into cells via binding to the binding site. However, if there are some cells capable of taking up and metabolizing L-glucose, they might be able to grow using it as an energy or carbon source with no need to compete for nutrient with normal cells. Thus, the uptake of L-glucose into cells can be used as a key measure for identifying cancer cells that can survive in a harsh environment by using the nutrient not available for normal cells and have a high metastasis potential. In fact, some cancer cells from patients with invasive cancer or poor postoperative outcomes have been found to take up L-glucose (Yamada, K. et al., US10551387B2; EP3199638B1; and Japanese Patent Nos. 6670503 and 6406715).

However, there have been no cases where L-glucose-cultured tumor cells are transplanted into animals, and it has been remained completely unclear what properties such cells can exhibit in vivo. The inventors have cultured tumor cells using a liquid medium containing a certain amount of L-glucose in place of the same amount of D-glucose. After suspending, in a saline, the cells surviving the culture conditions, the inventors have transplanted them into subcutaneous sites of posterior limb or intraperitoneal sites in immunodeficient mice without using any cofactor such as Matrigel. In fact, before the experiment, the inventors considered that transplantation of cultured cells without the use of Matrigel would fail to attain engraftment in the animal body. Initially, the subcutaneously transplanted L-glucose-cultured cells did not grow in the subcutaneous site, and the inventors considered that tumor cell engraftment was failed. However, the inventors found a decrease in the animals' blood sugar level and assumed that some tumor cells should exist somewhere. The inventors looked for such cells by a whole body searching process including: fixing the animal with formalin; and then sectioning the whole body at intervales of 5 mm to prepare pathological samples, and consequently observed sarcomatous lesions in the muscle, where tumor cells invaded deeply. Such a phenomenon was unexpected finding. The sarcomas exhibited functional activity and showed a metastatic potential comparable to a clinical manifestation. The phenomenon in which subcutaneously transplanted tumor cell lines deeply invade the muscle and form sarcoma is the first time, and when transplanted into the abdominal cavity, such tumor cells were found to similarly invade the muscle. In addition, clear metastatic manifestations such as lymph node metastasis and liver metastasis were also observed.

### Cancer Model Animal

Examples of animals that may be used for the cancer model animal according to the present invention include mice, rats, hamsters, guinea pigs, monkeys, cattle, pigs, horses, rabbits, sheep, goats, cats, dogs, and other non-human animals. Preferred examples of non-human animals include, but are not limited to, T cell function-deficient nude mice, such as BALB/c-nu/nu and F344/N-rnu/rnu, T- and B-cell-deficient mice, such as NOD/SCID mice, and other mice with mild immunodeficiency. Examples also include immunodeficient animals, such as NSG mice, nude rats, and ALY mice, and their modified strains.

Examples of tumor cells that may be used for the cancer model animal of the present invention include, but are not limited to, adenocarcinoma cells, such as lung cancer-derived tumor cells and neuroendocrine tumor cells, such as insulinoma cells. Examples of cancers from which the tumor cells may be derived include, but are not limited to, adenocarcinomas, such as lung cancer, pancreatic cancer, brain tumors, breast cancer, stomach cancer, gastroesophageal junction cancer, oral cancer, bile duct cancer, ovarian cancer, and endometrial cancer, and other various cancers, such as sarcomas and osteosarcomas. Examples of animal species from which the tumor cells may be derived include, but are not limited to, mice and humans.

A first embodiment of the present invention is directed to a cancer model animal comprising subcutaneously or intraperitoneally transplanted tumor cells that proliferate by culturing with an L-glucose-containing medium.

A second embodiment of the present invention is directed to a cancer model animal that is a sarcoma model animal comprising subcutaneously or intraperitoneally transplanted tumor cells invading a muscle. Examples of the muscle include skeletal muscle and smooth muscle. The sarcoma model animal in which the muscle is a skeletal muscle is unprecedented and particularly useful. In an example of the second embodiment of the present invention, the sarcoma model animal has subcutaneously transplanted tumor cells that do not swell on site but invade its skeletal muscle.

A third embodiment of the present invention is directed to a cancer model animal comprising subcutaneously or intraperitoneally transplanted tumor cells showing metastasis, wherein the tumor cells are selected from the group consisting of adenocarcinoma cells, neuroendocrine tumor cells, and sarcoma cells. As used herein, the term "metastasis" means that tumor cells have invaded from the transplantation site into any other site or have spread from the transplantation site and grown on any other site. Examples of metastasis cases include cases where intraperitoneally transplanted tumor cells have spread from the abdominal cavity to exhibit lymph node metastasis or peritoneal invasion, cases where transplanted tumor cells have metastasized to skeletal muscles such as abdominal muscles or smooth muscles to form sarcomas, and cases where transplanted tumor cells have metastasized to an intra-abdominal organ. As used therein, the term "intra-abdominal organ" is intended to include liver, spleen, stomach, intestine, pancreas, gallbladder, kidney, and adrenal gland.

In an example of the third embodiment of the present invention, the tumor cells are adenocarcinoma cells, and the metastasis is metastasis to the lymph node, the lung, or the intra-abdominal organ or accumulation of the tumor cells in the renal hilum. The adenocarcinoma cells are typically, but not limited to, lung cancer-derived tumor cells. The intra-abdominal organ is typically, but not limited to, the liver or spleen.

In another example of the third embodiment of the present invention, the tumor cells are neuroendocrine tumor cells, and the metastasis is metastasis to the lymph node, the intra-abdominal organ, or the peritoneal membrane. The neuroendocrine tumor cells are typically, but not limited to, insulinoma cells. The intra-abdominal organ is typically, but not limited to, the liver.

A fourth embodiment of the present invention is directed to a cancer model animal comprising subcutaneously or intraperitoneally transplanted tumor cells showing anomaly in orthotopic cell function, wherein the tumor cells are selected from the group consisting of adenocarcinoma cells, neuroendocrine tumor cells, and sarcoma cells. In an example of the fourth embodiment of the present invention, the adenocarcinoma cells are lung cancer-derived tumor cells. In another example of the fourth embodiment of the present invention, the neuroendocrine tumor cells are insulinoma cells. In a further example of the fourth embodiment of the present invention, the tumor cells show anomaly in orthotopic cell function within 3 days after transplantation, preferably within 2 days after transplantation, even more preferably within 1 day after transplantation, furthermore preferably in 1 day after transplantation. In a still further example of the fourth embodiment of the present invention, the tumor cells show anomaly in orthotopic cell function when the number of the tumor cells is 5.0 × 10⁵ or less, preferably 4.0 × 10⁵ or less, more preferably 3.0 × 10⁵ or less, even more preferably 2.0 × 10⁵ or less. The number of the tumor cells may have any lower limit. The tumor cells may show anomaly in orthotopic cell function when the number of them is 1.0 × 10⁵ or more.

In the fourth embodiment of the present invention, for example, when the tumor cells are neuroendocrine tumor cells and the neuroendocrine tumor cells are insulinoma cells, the cancer model animal can show a strong decrease in blood sugar level, caused by excessive insulin secretion, on day 1 after transplantation.

There are a wide variety of human sarcomas, and cancer cells capable of forming sarcomas can be derived from various organs. In the cancer model animal according to the fourth embodiment of the present invention, the subcutaneously or intraperitoneally transplanted adenocarcinoma cells (e.g., lung cancer-derived tumor cells) or neuroendocrine tumor cells (e.g., insulinoma cells) can deeply invade the muscle to form a sarcoma and can show the function of the organ from which the tumor cells have been derived (e.g., can show a strong decrease in blood sugar level in the case where the transplanted cells are insulinoma cells).

In a preferred embodiment of the present invention, the cells may be modified cells having a fluorescent or luminescent protein-encoding gene that has been operably integrated in the genome so as to express the protein stably. In the model animal created by the inventive preparation method using such cells, the cancer cell behavior such as growth, migration, or metastasis can be observed non-invasively or in real time in vivo using the fluorescence or luminescence as an indicator. The fluorescent protein may be Green Fluorescent Protein or any other suitable protein generally used in the art, and the luminescent protein may be any of various luciferases.

In the present invention, the cells are cultured using a medium containing L-glucose instead of D-glucose. As used herein, the term "L-glucose medium" refers to a medium that contains L-glucose and is substantially free of D-glucose or refers to a glucose-containing medium in which the glucose component consists essentially of L-glucose. For example, a serum-containing medium may be used. In such a case, the serum may contain a small amount of D-glucose, and the medium may contain a small amount of D-glucose (e.g., a medium containing 10% of Defined Fetal Bovine Serum (a typical serum preparation manufactured by Hyclone) may contain at most about 100 mg/L of D-glucose). Such a serum-containing medium is also encompassed by the term "L-glucose medium" as long as its glucose component consists essentially of L-glucose. The L-glucose medium for use in the present invention is a glucose-containing medium in which the glucose component consists essentially of L-glucose or in which L-glucose preferably makes up 98% or more, more preferably 99% or more of the glucose component. The L-glucose medium for use in the present invention may have any suitable L-glucose concentration. For example, the L-glucose medium may have the same concentration of L-glucose as that of D-glucose in the corresponding normal culture medium. The L-glucose concentration of the medium is typically, but not limited to, 0.5 mM to 50 mM, preferably 1.0 mM to 25 mM, more preferably 5 mM to 25 mM.

The L-glucose medium may be any type used for common cell culture as long as its glucose component consists essentially of L-glucose. Examples of the L-glucose medium include BME media, BGjB media, CMRL-1066 media, Glasgow MEM media, Improved MEM media, IMDM media, Medium 199, Eagles MEM media, αMEM media, DMEM media, Ham's media, RPMI 1640 media, Fischer's media, and any mixture thereof. The L-glucose medium may be any medium usable for animal cell culture. The L-glucose medium is preferably a DMEM medium. These media are commercially and readily available. The serum may be any commercially available serum used for standard culture. For example, good results can be obtained using serum from Hyclone.

In one aspect of the present invention, D-glucose may be removed from a medium, and the cells may be cultured using the L-glucose medium instead. A D-glucose medium may be changed to the L-glucose medium. It is important to start the change to the L-glucose medium after an appropriate culture duration depending on the cell type. The method for the change to the L-glucose medium may be changed as appropriate depending on the cell type. Examples of the method for the change include a method of replacing the D-glucose medium by the L-glucose medium and a method of gradually increasing the content of L-glucose in all glucose components. As a non-limiting example, the method for the change to the L-glucose medium may include repeating, appropriate times, the replacement of half the amount of the medium by the L-glucose-containing medium, so that the D-glucose concentration can be gradually reduced and the L-glucose concentration can be easily increased. As a non-limiting example, the replacement of half the amount may be performed on day 6 or 8 after the start of the culture using D-glucose. On day 10, the whole amount of the culture medium may be discarded, and the cells may be washed with the L-glucose medium so that D-glucose is removed as much as possible, and finally the culture medium may be 100% L-glucose medium. As a non-limiting example, after the culture medium is changed to the 100% L-glucose medium, half the amount of the medium may be replaced every 6 hours over 1 to 2 days. If the number of cells is small, the subsequent medium change may be performed every few days, and if the number of cells is large, the subsequent medium change may be performed every day.

In one aspect of the present invention, the cells may be cultured for at least a certain number of days after the culture medium is changed to the medium containing 100% of L-glucose (containing 0% of D-glucose) as glucose. At least a certain number of days may be selected as appropriate depending on the type of the cells used. For example, the cells may be cultured with the L-glucose medium for 3 to 10 days after the change, and is typically cultured with the L-glucose medium for 3 days or more, preferably 10 days or more, more preferably 11 days or more, even more preferably 13 days or more.

Any suitable type of cells, such as animal-derived cancer cells or cancer patient-derived cancer cells, may be cultured using the present culture method.

According to the inventors' previous research, the present culture method is expected to be also possible using, instead of L-glucose, L-fructose which is less abundant in nature than D-fructose. That is, it is expected that the present culture method can be used to obtain not only tumor cells that proliferate by culturing with L-glucose-containing medium but also tumor cells that proliferate by culturing with L-fructose-containing medium. It is also expected that not only a cancer model animal comprising subcutaneously or intraperitoneally transplanted tumor cells that proliferate by culturing with L-glucose-containing medium but also a cancer model animal comprising subcutaneously or intraperitoneally transplanted tumor cells that proliferate by culturing with L-fructose-containing medium.

Hereinafter, the present method will be described with reference to examples using human lung cancer cells A549. It will be understood by those skilled in the art, however, that other cells can be used for the present method. Those skilled in the art can use such other cells according to known information about each type of cells under appropriately selected conditions, including materials and reagents.

Human lung cancer cells A549 are suspended in a commercially available D-glucose-containing medium to form a cell suspension. The cell suspension may be seeded on dishes or coverslips according to the result of the observation and analysis performed on the cell suspension. Any appropriate number of the cells may be seeded depending purpose. For example, the cells may be seeded at 3.0 to 6.0 × 10⁵/dish on commercially available 90 mm-diameter dishes (Greiner TC Petri Dish 664160) or at 1.0 × 10⁵/dish on WillCo-Glass bottom dishes (WillCo Wells 170 micron 40 mm in diameter).

Next, after a certain period of time after the start of culture, the D-glucose medium is changed to the L-glucose medium. The A549 cells may be cultured using the D-glucose medium for 1 day, and on day 2, the D-glucose medium may be changed to the L-glucose medium, with which the culture may be continued. The medium change may be performed using methods known to those skilled in the art. For example, the D-glucose medium may be removed from the cell culture, or the cells may be collected from the culture medium, and then the L-glucose medium may be added to the cells.

In a case where a sudden change in medium composition is undesirable, the following gradual process may be used. First, the medium change is performed until the medium D-glucose concentration reaches 25% (6.25 mM) of the initial concentration. To obtain 25% of the initial D-glucose concentration, half the amount of the medium may be changed twice consecutively. The medium change may be performed using a medium prepared by adding an appropriate amount of L-glucose to a D-glucose-free medium.

A suitable timing for the reduction of the D-glucose concentration to 25% of the initial concentration is 2 to 10 days after the start of culture. Subsequently, the cells may be cultured at the reduced D-glucose concentration (25% of the initial concentration) for 2 days and then subjected to a gradual concentration reduction process, which may include changing half the amount of the medium to reduce the D-glucose concentration to 12.5% (3.125 mM) of the initial concentration; culturing the cells at the reduced D-glucose concentration (3.125 mM) for 2 days; then discarding the whole amount of the medium; washing the cells with the L-glucose-containing medium twice; and then adding the L-glucose medium to the cells so that the medium D-glucose concentration can be reduced to 0%.

When cultured using the L-glucose medium, the A-549 cells become layered. The cells may be proliferated to a sufficient density for transplantation, then subjected to trypsin treatment according to standard procedures, and suspended in the L-glucose medium to form a cell suspension.

For example, the cells may be transplanted at 3 × 10⁶ cells/(100 µL)/animal into mice for tumor formation. Transplantation without any intercellular matrix such as Matrigel may result in a low engraftment rate. In general, therefore, a large number of cells should be transplanted for a high engraftment rate. However, the tumor cells cultured using the L-glucose medium can be transplanted at as low as 1.0 × 10⁵ cells/(20 µL)/animal for a high possibility of tumor formation as demonstrated later (see FIGS. 8 to 11).

The use of the L-glucose medium may lead to the following, although it depends on the cell type. During the culture, as the D-glucose concentration decreases from 12.5% (3.125 mM) of the initial concentration, dead floating cells become noticeable among the proliferating cells, and after the D-glucose concentration reaches 0%, floating cells become prominent and the number of the cells, which has increased before, drops temporarily. From the time point when the medium D-glucose concentration reaches 0%, half the amount of the medium may be changed to the L-glucose-containing medium every 6 hours so that the metabolic products from the dead floating cells are removed. This process can minimize the influence of the metabolic products. The set of medium changes every 6 hours may be performed at least 4 times, preferably 8 times or more, until the floating cells are no longer noticeable. Subsequently, the medium change is preferably performed every other day during a period when the number of the cells is relatively small over the dish, and on and after day 19, when the number of the cells begins to increase to a sufficient level, half the amount of the medium is preferably changed daily.

### Cell Composition for Use in Creation of Cancer Model Animal

The present invention is directed to a cell composition comprising tumor cells that proliferate by culturing with L-glucose-containing medium, wherein the cell composition is subcutaneously or intraperitoneally transplanted into an animal and is used to create a cancer model animal. The animal, tumor cells, and other conditions are as described above. The cell composition of the present invention may contain the medium described above, such as the L-glucose medium, the serum described above, a solvent, such as water, a physiologically acceptable salt, and any other optical component.

### Method for Creating Cancer Model Animal

The present invention is directed to a method for creating a cancer model animal, comprising subcutaneously or intraperitoneally transplanting the cell composition of the present invention into an animal. A single type of the cell composition or a combination of two or more types of the cell compositions may be used depending on the cancer model animal to be created. The animal may be an adult. For example, the animal may be a 5 to 9 week old mouse or a 6 to 10 week old rat. Any suitable method may be used to subcutaneously or intraperitoneally transplant the cell composition into an animal. For example, an injection syringe with a needle may be used to transplant the cell composition into the subcutaneous or intraperitoneal site of the animal. After an appropriate period of time after transplantation (e.g., 4 to 5 or more days after transplantation), the presence or absence of tumor formation in the animal may be determined for the determination of whether the cancer model animal has been crated. The determination may be performed by measuring various physiological functions, by dissecting the animal, or by non-invasively observing, as an indicator, fluorescence or luminescence from a fluorescent or luminescent protein that is encoded by a gene operably integrated in the cell genome.

### Method of Screening Substances that Have Anticancer Effect

The present invention is directed to a method of screening substances that have anticancer effect, comprising:
a step of administering a test substance to the model animal of the present invention;
a step of evaluating tumor cell growth or tumor cell metastasis after starting the administration of the test substance; and
a step of comparing a result of the evaluation in the evaluating step with a result of evaluation of tumor cell growth or tumor cell metastasis in a model animal to which the test substance was not administered.

As used herein, the term "anticancer substance" refers to a substance that is effective in inhibiting the growth of tumor cells.

Examples of the test substance include, but are not limited to, sugars, nucleic acids, peptides, proteins, cell extracts, cell culture supernatants, plant extracts, mammalian tissue extracts, plasma, and so on. The test substance may form a salt. The test substance salt may be a salt with a physiologically acceptable acid or with a physiologically acceptable base. The test substance may be administered in any suitable amount, which may be selected as appropriate based on published literatures or preliminary tests. The route of administration may be selected as appropriate depending on the test substance. For example, the route of administration may be oral, intravenous, intraperitoneal, subcutaneous, intramuscular, intratumoral, or any other route known in the art. The administration may be performed for any suitable period and at any suitable intervals, which may be selected as appropriate depending on the test substance.

The tumor cell growth or metastasis may be evaluated at any timing after the start of administration of the test substance. The tumor cell growth may be evaluated by visual observation, if possible, and measurement of size, weight, or other visible features or by pathological autopsy. The tumor cell growth may also be evaluated using any of various fluorescent probes, such as 2-[N-(7-nitrobenz-2-oxa-1,3-diazol-4-yl)amino]-2-deoxy-L-glucose (2-NBDLG) (a fluorescent L-glucose derivative having a fluorescent group NBD introduced at the 2-position of L-glucose) or 2-[N-(7-nitrobenz-2-oxa-1,3-diazol-4-yl)amino]-2-deoxy-D-glucose (2-NBDG) (a D-glucose derivative (an enantiomer of the L-glucose derivative)) and a whole-body imaging system, a fluorescent endoscope, or a laparoscope. Alternatively, the tumor cell growth may be evaluated using a luminescence imaging technique that includes transplanting luciferase-transfected cells and detecting the luminescence using any suitable luminescence probe such as luciferin or evaluated using animal X-ray CT, animal magnetic resonance imaging (MRI), or similar techniques. The tumor cell growth may also be evaluated by positron emission tomography using a radioactive labeled probe such as ¹⁸F-2-deoxy-D-glucose (FDG). The tumor cell metastasis may also be evaluated using visual observation, if possible, and measurement of size, weight, or other visible features, using pathological autopsy, or using in vivo imaging by fluorescence detection, luminescence detection, X-ray CT, MRI, or PET.

The result of evaluation in the evaluating step is compared with the result of evaluation of tumor cell growth or metastasis in the model animal not administered with the test substance. The test substance may be determined to have anticancer activity when the former evaluation result is significantly lower in tumor cell growth or metastasis than the latter evaluation result.

### EXAMPLES

Hereinafter, the present invention will be described in more detail with reference to examples, which are not intended to limit the present invention at all.

### Cancer Model Animal Created Using Human Lung Cancer-Derived Tumor Cells

Human lung cancer-derived tumor cells A549 cultured with L-glucose were ectopically transplanted into the abdominal cavity without using Matrigel. The animals used were BALB/c nu/nu (nude mice). They were purchased at 5 to 7 week old and housed. Luciferase-transduced tumor cells were transplanted into some of the mice. After the transplantation, the tumor growth was observed using whole-body imaging in the case of the luciferase-transduced tumor cell transplantation and, in the other case, observed using a different whole-body imaging method such as CT or MRI. Thereafter, some of the mice were anesthetized and then subjected to whole-body fixation with 10% neutral buffered formalin. Cell transplantation site samples, hindlimb muscle samples (control samples opposite the transplantation site), and organs were harvested from some of the mice and fixed with 10% neutral buffered formalin. The fixed whole body was sectioned at intervals of 5 mm for the preparation of paraffin-embedded section samples. The samples were subjected to HE staining and immunostaining with antibodies for pathological examination according to normal procedures. As a result, the human lung cancer-derived tumor cells A549 were found to invade the abdominal muscle and form sarcoma (FIG. 1A). The enlarged image (FIG. 1B) shows a clear manifestation of invasive cancer integrated into the abdominal muscle. This result indicates that the tumor cells cultured with the L-glucose medium have a high ability to invade the muscle, namely sarcoma-forming ability. To the best of the inventors' knowledge, there has been no model having intraperitoneally ectopically transplanted tumor cell lines invading deeply abdominal muscles and forming highly atypical sarcoma.

In the present model, the tumor cells A549 cultured with L-glucose significantly invaded different intra-abdominal organs (e.g., liver metastasis (FIG. 2A), lymph node metastasis (FIG. 2B), spleen metastasis (FIG. 2C)). The present model clearly showed cancer metastasis (FIG. 2D).

It is important to examine the impact of the ectopic transplantation of the human lung cancer-derived tumor cells A549 on the respiratory function and kidney functions involved in cardiovascular regulation in coordination with the lung, such as the body's pH control. As shown in FIG. 3, the pathological autopsy of the mouse having intraperitoneally-transplanted (without Matrigel), L-glucose-cultured, human lung cancer-derived tumor cells A549 revealed invasion of cancer into the lung.

As shown in FIG. 3, after the intraperitoneal transplantation of the human lung-derived tumor cells A549 obtained by culturing with the L-glucose medium, the resulting cancer invaded and settled in the mouse lung, which was associated with vasculogenesis. Since the lesion areas had evident characteristics of cancer, the mice were euthanized for animal welfare reasons at this time. However, if allowed to stand, it would surely impair the normal lung respiratory function.

The present model also exhibited a remarkable change in kidney function, which has not been previously reported as far as the inventors know with respect to animal models. The lung is known as the most common primary site for metastasis of human kidney cancer. On the other hand, some literatures reported cases where human lung cancer patients showed kidney metastasis of lung cancer, in many of which the metastasis was not detected in clinical examination during life but detected for the first time by autopsy. As shown in FIGS. 4A and 4B, the mouse kidney having intraperitoneally-transplanted human lung cancer-derived tumor cells A549 obtained by culturing with L-glucose showed formation of a giant tumor thrombus, which is not usually observable in the renal hilum of the left or right kidney.

The thrombus showed coagulative necrosis of nucleated cells distinct from red blood cells, and the image information suggested that the tumor thrombus was caused by high accumulation of the transplanted human lung cancer cells A549 in the renal hilum vascular structures. In general, when malignant tumors invade blood vessels, thrombosis is prone to occur due to coagulation anomalies. However, the formation of such a giant thrombus is not normal. Distant metastasis of cancer to other organs requires the success of all of many steps in which cancer cells must migrate from the primary lesion through vascular channels such as blood and lymph vessels and settle in the metastatic lesion. It is a particularly big point in the distant metastasis process whether and how cancer cells can undergo extravasation and invade target organ tissues even when they successfully undergo intravasation from the primary lesion, migrate in the living state through blood circulation, and arrive at the blood vessels in the target organ. In such a field, there are various theories and no conclusion has been reached. In the model animal according to the present invention, a large number of human cancer-derived tumor cells A549 can successfully show an intermediate state in which they still remain not invading the kidney tissues while highly accumulating in thick veins of the renal hilum of the kidney. Thus, the model animal according to the present invention provides a model valuable for the study of the process for kidney metastasis of lung cancer cells (Stebbing, J. and Smith, I. E. Clinical Oncology 12: 326-327, 2000), the study of the process for distant metastasis of other various cancers to other organs, and the development of various anticancer agents for preventing distant metastasis with extremely low survival rates.

The kidneys are organs that continuously filter a large volume of blood, excrete toxins and unnecessary waste products into urine, and reabsorb essential nutrients and ions back into the bloodstream via the renal tubules. The formation of the giant thrombus almost blocking the blood flow in the renal hilum suggested that chronic kidney diseases with severe damage to the normal kidney function occurred like those observed for human malignant kidney tumors.

Cancer Model Animal Created Using Mouse Insulinoma Cells Not only human cancer cells A549 but also mouse insulinoma cells MIN6 can be successfully cultured with the L-glucose medium. Mouse insulinoma cells MIN6 were cultured with the D-glucose medium. At a stage where the cells proliferated sufficiently, the medium was changed to the L-glucose medium. Subsequently, the medium change was performed frequently over 2 to 3 days. After almost all dead cells were confirmed to be removed, half the amount of the medium was changed every other day. The MIN6 cells were passaged once or twice using the L-glucose medium. After the culture for 8 to 11 days, the MIN6 cells were trypsinized to form a cell suspension, which was transplanted at 5 × 10⁶ cells (100 µL)/mouse subcutaneously into the right thigh of mice or at 1.0 × 10⁵ cells (20 µL)/mouse into the abdominal cavity of mice without using Matrigel. As a control, MIN6 cells cultured with the normal D-glucose medium were also transplanted into mice. Because of rapid growth in the D-glucose medium, MIN6 cells were cultured with the D-glucose medium for 6 to 7 days and then transplanted at the same density as that of L-glucose-cultured cells subcutaneously into the right thigh of mice.

After being cultured with the L-glucose medium, the MIN6 cells were subcutaneously transplanted into mice. However, unlike common subcutaneous transplantation mouse models, the mice with the transplanted MIN6 cells did not show external signs of tumor (FIG. 5). Thus, pathological autopsy was performed on the mice. As a result, it was revealed that instead of growing in the subcutaneous site, the tumor cells invaded deeply a central portion of a thigh skeletal muscle layer under the subcutaneous site and formed malignant tumors (namely sarcomas) in the muscle (FIGS. 6A and 6B). Standard HE staining and pathological specialist physician's examination revealed that the cells showed a strong atypia, aggressive malignant tumor features with significant angiogenesis, and formation of highly malignant sarcoma (FIG. 6B).

Many cell-line-derived subcutaneous tumor models are available worldwide. Unfortunately, despite the urgent need for a good animal model for sarcomas, such a model has not yet been established. In fact, to the best inventors' knowledge, there has been no known model in which tumor cell lines transplanted ectopically into subcutaneous sites deeply invade the skeletal muscle immediately under the subcutaneous sites, instead of settling therein, and form highly atypical sarcoma. In particular, the resulting sarcoma cells not only invaded the muscle but also caused the animal to show a clear reduction in blood sugar level, caused by excessive insulin secretion (their functional characteristics) like human insulinoma cells. The mice having transplanted, L-glucose medium-cultured MIN6 cells all showed a significant reduction in blood sugar level from day 1 after transplantation (the filled triangle in FIG. 7) as compared with those having transplanted, normally-D-glucose medium-cultured MIN6 cells (the filled circle in FIG. 7) or as compared with the negative control (the hollow circle in FIG. 7) (a significant difference was found between the control group and the L-glucose group and between the D-glucose group and the L-glucose group in the ANOVA and Bonferroni's multiple comparison test).

The transplantation of the L-glucose-cultured insulinoma cells into the mouse abdominal cavity resulted in not only the settlement of the cells in the abdominal cavity (FIGS. 8A and 8B) but also the deep invasion of the cells into the abdominal muscle (FIGS. 9A and 9B). The result indicates that the tumor cells cultured with the L-glucose medium have a high ability to invade the muscle (namely an ability to form sarcoma).

Surprisingly, the intraperitoneally transplanted mouse insulinoma cells also showed lymph node metastasis (FIGS. 10A and 10B) and liver metastasis (FIGS. 11A and 11B), which are frequently observable for neuroendocrine tumors such as human insulinoma. The histopathological malignant features of the liver metastasis were so similar to those of human neuroendocrine tumors that they could have been easily mistaken for such human tumors (FIG. 11B) if the situation was not recognized.

Insulinoma (also known as pancreatic neuroendocrine tumor (PNET)) is a type of neuroendocrine tumor (NET). Among NETs, insulinoma is reported to have the highest rate of distant metastasis (40.3% in the United State) already at the time of diagnosis, and in such distant metastasis cases, the survival period is only 12 months. It is also reported that the five-year survival rate of insulinoma is 22.7%. Despite its extremely poor prognosis, many aspects of this rare disease remain unclear. Therefore, the animal model according to the present invention should be a unique valuable model that is expected to contribute to the medical understanding of PNET-derived sarcomas, the development of therapy for PNET-derived sarcomas, and the diagnosis and treatment of human malignant insulinoma (i.e., metastatic PNET).

### Examination of Method of Screening Substances that Have Anticancer Effect

A method of screening substances that have anticancer effect was examined using, as a test substance, 2-deoxy-2-(2-oxo-2H-chromen-7-yl)amino-L-glucose (hereinafter referred to as "CLG") (Japanese Patent No. 6566348), which is a molecule having a 7-OH-cuoumarin moiety bonded to L-glucose via a nitrogen atom.

On day 0, insulinoma cells MIN6 cultured with L-glucose were intraperitoneally transplanted into 32 mice (four groups each including 8 mice). On day 1, the following groups were prepared:
- 8 mice that were intraperitoneally administered every day until day 4 (over 4 minimum days) with a saline solution of CLG at a concentration of 53 mg/kg expected to be safe (CLG administration group);
- 8 mice that were intraperitoneally administered only with a CLG-free saline every day until day 7 (over 7 days) (saline administration group); and
- 8 mice that were intraperitoneally administered every day until day 7 (over 7 days) with 5-FU (which is used as a standard treatment for pancreatic cancer in Japan and the Europe and also used for treatment of neuroendocrine tumors such as insulinoma) at a high concentration of 25 mg/kg acceptable for nude mice (5-FU administration group).

All mice were euthanized at month 5 and subjected to pathological autopsy.

In the CLG administration group, 4 of the 8 mice maintained normal blood sugar levels until the final euthanasia on month 5. No tumor was observed in the dissected 4 mice. Among the 8 mice, the remaining 4 mice began to show a reduction in blood sugar level one week later than some of the 8 mice in the saline administration group began to show it. After each of the 4 mice was fixed with formalin, the whole body was cut into ring sections at intervals of 5 mm, and each section was subjected to detailed pathological examination. As a result, one of the mice was found to have no tumor. Another mouse was found to have small tumor cells, which were well-differentiated with abundant cytoplasm, although having varying nuclear sizes, were not high in N/C ratio, were not visible without a microscope, and were present, being covered with a fibrous membrane, not in the abdominal cavity but in subcutaneous fatty tissue outside the peritoneal membrane. Still another mouse was found to have small, round, low-grade tumor cells with low malignancy and visible cytoplasm, which were encapsulated and well-differentiated, had uniform nuclei with no strong atypia, and were present not in the abdominal cavity but in the subcutaneous site outside the peritoneal membrane. The position and structural features of the tumors in these two mice suggested that they were all completely removable by surgery. Only one remaining mouse was found to have, in the abdominal cavity, a malignant tumor classifiable as neuroendocrine carcinoma (NEC). However, at least 90% of the intraperitoneal tumor had undergone necrosis. In this mouse, metastasis was also observed within the abdominal muscle; however, the tumor cells exhibited a compartmentalized growth pattern, which was distinct from the cancer-like tissue morphological features in other groups.

In summary, among the 8 mice in the CLG administration group, 4 mice (50%) had normal blood sugar levels and no tumor, and among the remaining 4 mice, one had no tumor although it showed a reduction in blood sugar level, another one had a benign tumor in subcutaneous tissue outside the peritoneal membrane (transplantation site), still another one had a low-malignancy tumor also in subcutaneous tissue outside the peritoneal membrane, and the remaining one had a malignant tumor associated with necrosis.

Thus, the CLG administered only for 4 days had a significant effect in vivo. The in vivo result was as expected from in vitro experiments performed separately on the anticancer effect of CLG.

In the saline administration group, one mouse was removed because of infection after the administration, but the remaining mice were all subjected to analysis. Among the remaining 7 mice, 4 mice had obvious tumors. Specifically, one mouse was found to have a highly-atypical, high-grade sarcoma invading the abdominal wall muscle, another mouse was found to have clear invasion into the abdominal muscle, and still another mouse was found to show a reduction in blood sugar level and have a highly malignant tumor in the abdominal cavity. Yet another mouse was found to show a reduction in blood sugar level and have a highly-atypical, large neuroendocrine tumor that was easily identifiable as malignant and metastasized to the lymph node. The remaining one mouse was found to have a lesion suspicious for neuroendocrine tumor in the abdominal cavity. In summary, among the 7 mice analyzed successfully, 4 mice had a metastatic malignant tumor, one mouse had a tumor, and the remaining two mice had no tumor.

Among the 8 mice in the 5-FU administration group, shortly after day 84, one mouse exhibited a low blood sugar level and was found to have large, highly-atypical, significant liver metastasis with high malignancy, in which the primary lesion adhered to the surface of the seminal vesicle. Since liver metastasis of neuroendocrine tumors are also observed in humans, this model with similar features should be useful. One mouse was found to have invasive cancer in the smooth muscle around the transverse colon (neuroendocrine tumor) (FIGS. 12A and 12B). On day 10, 3 other mice were likely to have worsen conditions and thus subjected to dissection. Among the 3 mice, one was found to have a tumor on the retroperitoneal surface, another was found to have a tumor on the peritoneal surface, and another was found to have a tumor in the abdominal cavity between the retroperitoneum and the intestine, which is not normally seen in mice. Immunostaining with chromogranin antibody, synaptophysin antibody, and insulin antibody showed that all of the tumors were neuroendocrine tumors and that the tumors settled even after only a short period after the MIN6 transplantation.

The results show that the cancer model animal according to the present invention can be very useful to screen substances that have anticancer effect. The cancer model animal according to the present invention, which can exhibit functional expressions at an early stage after transplantation, is also expected to be useful for the development of various methods for early detection of cancer.

## Claims

1. A cancer model animal comprising subcutaneously or intraperitoneally transplanted tumor cells that proliferate by culturing with an L-glucose-containing medium.

2. A sarcoma model animal comprising subcutaneously or intraperitoneally transplanted tumor cells invading a muscle.

3. The sarcoma model animal according to claim 2, wherein the muscle is a skeletal muscle.

4. A caner model animal comprising subcutaneously or intraperitoneally transplanted tumor cells showing metastasis, wherein the tumor cells are selected from the group consisting of adenocarcinoma cells, neuroendocrine tumor cells, and sarcoma cells.

5. The cancer model animal according to claim 4, wherein the tumor cells are adenocarcinoma cells, and wherein the metastasis is metastasis to a lymph node, a lung, or an intra-abdominal organ or accumulation in a renal hilum.

6. The cancer model animal according to claim 5, wherein the adenocarcinoma cells are lung cancer-derived tumor cells.

7. The cancer model animal according to claim 5, wherein the intra-abdominal organ is liver or spleen.

8. The cancer model animal according to claim 4, wherein the tumor cells are neuroendocrine tumor cells and wherein the metastasis is metastasis to a lymph node, an intra-abdominal organ, or a peritoneal membrane.

9. The cancer model animal according to claim 8, wherein the neuroendocrine tumor cells are insulinoma cells.

10. The cancer model animal according to claim 8, wherein the intra-abdominal organ is liver.

11. A cancer model animal comprising subcutaneously or intraperitoneally transplanted tumor cells showing anomaly in orthotopic cell function, wherein the tumor cells are selected from the group consisting of adenocarcinoma cells, neuroendocrine tumor cells, and sarcoma cells.

12. The cancer model animal according to claim 11, wherein the adenocarcinoma cells are lung cancer-derived tumor cells.

13. The cancer model animal according to claim 11, wherein the neuroendocrine tumor cells are insulinoma cells.

14. The cancer model animal according to claim 11, wherein the tumor cells show anomaly in orthotopic cell function within 3 days after transplantation.

15. The cancer model animal according to claim 11, wherein the tumor cells show anomaly in orthotopic cell function when the number of the tumor cells is 5.0 × 10⁵ or less.

16. A cell composition comprising tumor cells that proliferate by culturing with an L-glucose-containing medium, wherein
the cell composition is subcutaneously or intraperitoneally transplanted into an animal and is used to create a cancer model animal.

17. A method for creating a cancer model animal, the method comprising subcutaneously or intraperitoneally transplanting the cell composition according to claim 16 into an animal.

18. A method of screening substances that have anticancer effect, the method comprising:
a step of administering a test substance to the cancer model animal according to any one of claims 1 to 15;
a step of evaluating tumor cell growth or tumor cell metastasis after starting the administration of the test substance; and
a step of comparing a result of the evaluation in the evaluating step with a result of evaluation of tumor cell growth or tumor cell metastasis in a model animal to which the test substance was not administered.
